# FASCICULE DE BREVET EUROPEEN

(11) **EP 3 192 434 B1**
(45) Date de publication et mention de la délivrance du brevet: **03.10.2018**
(21) Numéro de dépôt: 16305042.0
(22) Date de dépôt: 15.01.2016
(51) Int. Cl.: A61B 3/113, A61B 3/024, G06F 3/01

(54) **DISPOSITIF ET PROCÉDÉ DE DÉTERMINATION DES MOUVEMENTS OCULAIRES PAR INTERFACE TACTILE**
VORRICHTUNG UND VERFAHREN ZUR BESTIMMUNG DER AUGENBEWEGUNG DURCH BERÜHRUNGSEMPFINDLICHE SCHNITTSTELLE
DEVICE AND METHOD FOR DETERMINING EYE MOVEMENTS BY TOUCH INTERFACE

(43) Date de publication de la demande: 19.07.2017
(73) Titulaire: Centre National de la Recherche Scientifique, 75016 Paris (FR); Université Claude Bernard Lyon 1, 69100 Villeurbanne (FR)
(72) Inventeur: SIRIGU, Angela, 75016 PARIS (FR); DUHAMEL, Jean-René, 75016 PARIS (FR); LIO, Guillaume, 75016 PARIS (FR)
(74) Mandataire: GIE Innovation Competence Group

(56) Documents cités:
- US-A- 5 668 622
- US-A1- 2011 205 167
- US-A1- 2011 270 123

## Description

L'invention concerne le suivi des mouvements oculaires, et en particulier les dispositifs destinés à enregistrer avec précision les mouvements oculaires.

L'oeil humain explore l'espace visuel au moyen de déplacements rapides du regard, appelés saccades oculaires, qui alternent avec des périodes de fixation du regard plus ou moins longues. Ces mouvements sont nécessaires car à un instant donné, l'image rétinienne n'est nette que sur une plage de quelques degrés d'angle visuel, correspondant à la région centrale de la rétine, appelée fovéa. La sensation subjective de disposer d'un large champ visuel, coloré et détaillé, d'un angle d'environ 120°, résulte en réalité d'un processus de construction opéré au niveau cortical, intégrant les vues successives obtenues au moyen de séquences d'exploration oculaire ou visuelle.

L'exploration visuelle est une tache naturelle et non contraignante effectuée en permanence par l'oeil humain. L'exploration visuelle optimisée implique un grand nombre de capacités perceptives, motrices et cognitives. Ainsi, l'enregistrement, la quantification et l'analyse des comportements oculaires est une méthode particulièrement adaptée pour la détection d'une pathologie, du fait d'une exploration visuelle caractéristique de cette pathologie.

A titre d'exemple :
- un comportement d'évitement du regard peut être caractéristique de troubles du spectre autistique ;
- une exploration visuelle focalisée sur la moitié d'une scène pourra indiquer une négligence spatiale unilatérale.

Ainsi, d'un point de vue clinique, l'oculométrie est une méthode à fort potentiel pour le diagnostic et le suivi de pathologies neuro-psychiatriques.

Au-delà du domaine médical, on constate également que la vision et le comportement d'observation sont généralement de bons moyens de prédire une action future. En effet, on observe souvent un objet avant de s'en saisir et, de même, on ne saisit pas un objet que l'on n'a pas vu. Ce simple exemple fait de l'oculométrie un outil très utilisé en ergonomie, pour l'optimisation des interfaces hommes-machines, des emballages de produit ou des documents publicitaires notamment.

Parmi les outils connus utilisés pour analyser de façon précise l'exploration visuelle par un observateur ou sujet, on peut citer les dispositifs d'oculométrie. Un dispositif d'oculométrie réalise usuellement l'enregistrement des mouvements oculaires d'un observateur en se basant sur un système incluant un afficheur, une ou plusieurs caméras haute vitesse, un ou plusieurs illuminateurs infrarouges, des processeurs de calcul et une suite logicielle adaptée. De tels dispositifs d'oculométrie réalisent des mesures précises de l'exploration oculaire mais présentent un coût d'acquisition et d'utilisation très élevé. De tels dispositifs d'oculométrie ne sont donc pas accessibles au grand public. Du fait de la limitation de l'accès à de tels dispositifs, les études peuvent difficilement être réalisées sur un nombre d'observateurs suffisant pour garantir leur fiabilité.

Le document US 2011/270123 révèle un dispositif de détection du mouvement des yeux comprenant un afficheur tactile, une mémoire numérique mémorisant des images d'angiographie CT, un processeur configuré pour déterminer les fixations des yeux et les saccades et pour élargir l'image en correspondance avec la direction de fixation.

US 2011/205167 révèle une tablette à afficheur tactile dans lequel l'utilisateur doit poursuivre une cible avec le doigt. Les position successives de la cible et du doigt sont enregistrées et comparées entre elles.

Par ailleurs, de tels dispositifs d'oculométrie nécessitent une utilisation dans un environnement contrôlé, avec des phases de calibration contraignantes pour les mouvements de l'observateur, ces conditions étant parfois incompatibles avec certaines pathologies des observateurs ou avec des observateurs présentant une moindre concentration tels que des enfants. L'observateur doit notamment le plus souvent être positionné à une distance contrôlée de l'afficheur, immobile et calé à l'aide d'une mentonnière orthoptique.

L'invention vise à résoudre un ou plusieurs de ces inconvénients. L'invention porte ainsi sur un dispositif pour la détermination de mouvements oculaires, comprenant :
- un afficheur tactile ;
- une mémoire numérique mémorisant une image incluant au moins un point d'intérêt ;
- un dispositif de traitement, configuré pour mettre en oeuvre une répétition d'étapes de :
   - détermination de la position d'un point de contact d'un utilisateur avec l'afficheur tactile et localisation de la position correspondante dans l'image en cours d'affichage sur l'afficheur tactile ;
   - génération d'une image dégradée à partir de l'image mémorisée, l'image dégradée étant :
      - sensiblement identique à l'image mémorisée dans une zone nette incluant la position du point de contact déterminée et ses alentours;
      - dégradée avec un paramètre de dégradation périphérique au-delà de la zone nette;
   - affichage sur l'afficheur tactile de l'image dégradée générée ;
   - l'enregistrement des positions successives du point de contact avec l'afficheur tactile.

L'invention porte également sur un procédé comprenant les étapes de :
- fourniture d'un dispositif incluant un afficheur tactile et une mémoire numérique mémorisant une image incluant au moins un point d'intérêt ;
- répétition d'étapes de :
- détermination de la position d'un point de contact d'un utilisateur avec l'afficheur tactile et localisation de la position correspondante dans une image en cours d'affichage sur l'afficheur tactile ;
- génération d'une image dégradée à partir de l'image mémorisée, l'image dégradée étant :
   - sensiblement identique à l'image mémorisée dans une zone nette incluant la position du point de contact déterminée et ses alentours ;
   - dégradée avec un paramètre de dégradation périphérique au-delà de la zone nette ;
- affichage sur l'afficheur tactile de l'image dégradée générée ;
- l'enregistrement des positions successives du point de contact avec l'afficheur tactile.

L'invention porte également sur les variantes suivantes. L'homme du métier comprendra que chacune des caractéristiques des variantes suivantes peut être combinée indépendamment aux caractéristiques ci-dessus, sans pour autant constituer une généralisation intermédiaire.

Selon une autre variante, ladite image dégradée est générée par application d'un floutage de l'image mémorisée au-delà de la zone nette.

Selon encore une variante, la génération de l'image dégradée à partir de l'image mémorisée est configurée pour qu'un indice SSIM moyen entre l'image mémorisée et l'image dégradée soit compris entre 0,4 et 0,8 et dans lequel le rapport entre la surface de la zone nette et la surface de l'image dégradée générée est compris entre 0,01 et 0,5.

Selon encore une autre variante, la génération de l'image dégradée à partir de l'image mémorisée est configurée pour qu'un indice SSIM moyen entre l'image mémorisée et l'image dégradée soit compris entre 0,4 et 0,7 et dans lequel le rapport entre la surface de la zone nette et la surface de l'image dégradée générée est compris entre 0,01 et 0,15.

Selon une variante, ladite zone nette inclut un cercle d'un diamètre d'au moins 8 mm au-dessus du point de contact.

Selon encore une variante, la position du point de contact de l'utilisateur avec l'afficheur tactile est échantillonnée avec une période comprise entre 10 et 50 ms.

Selon encore une autre variante, ladite répétition d'étapes est exécutée pendant une durée au moins égale à 5 secondes.

Selon une autre variante, l'image mémorisée illustre plusieurs êtres vivants formant chacun au moins un point d'intérêt.

Selon encore une variante, le procédé comprend :
- l'exécution d'un procédé de détermination de mouvements oculaires tel que décrit ci-dessus ;
- la comparaison entre les positions successives enregistrées du point de contact et une trajectoire d'exploration visuelle de référence ;
- la détermination d'une anomalie d'exploration visuelle lorsque les positions successives enregistrées du point de contact divergent de la trajectoire d'exploration visuelle de référence au-delà d'un certain seuil.

D'autres caractéristiques et avantages de l'invention ressortiront clairement de la description qui en est faite ci-après, à titre indicatif et nullement limitatif, en référence aux dessins annexés, dans lesquels :
- la figure 1 est une vue en coupe schématique d'un oeil humain ;
- la figure 2 est un diagramme d'acuité visuelle relative en fonction d'un angle par rapport à la fovéa ;
- les figures 3 à 6 illustrent une image et un exemple de mouvement oculaire en fonction de centres d'intérêt de l'image ;
- la figure 7 illustre un exemple de perception instantanée de l'image de la figure 3 lors de la focalisation sur un centre d'intérêt ;
- la figure 8 est une représentation schématique d'un dispositif de détermination des mouvements oculaires selon un exemple de mode de réalisation de l'invention ;
- la figure 9 est une représentation d'un exemple d'image mémorisée dans un dispositif de détermination des mouvements oculaires ;
- la figure 10 est une représentation d'une image totalement floutée générée à partir de l'image mémorisée de la figure 9 ;
- la figure 11 est une représentation d'un exemple d'image affichée en fonction du point de contact de l'utilisateur avec le dispositif de détermination des mouvements oculaires ;
- la figure 12 est un exemple d'image dégradée générée à partir d'une image contenant des informations alphanumériques ;
- la figure 13 est un diagramme illustrant des paramètres garantissant une exploration oculaire optimale par l'intermédiaire du dispositif.

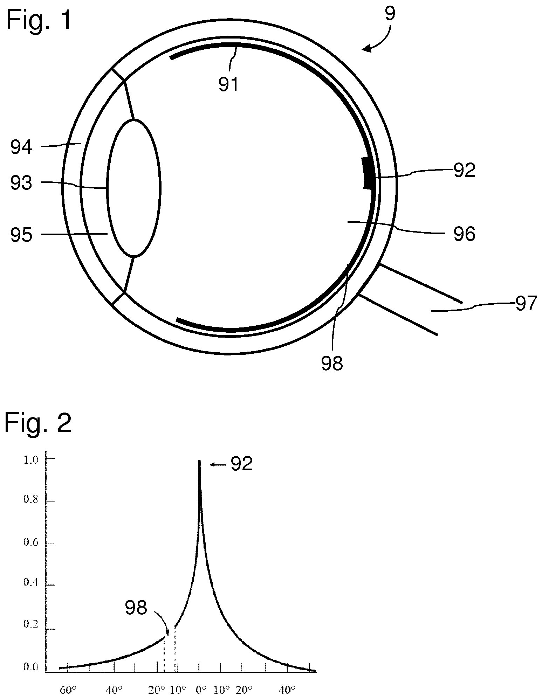

La figure 1 est une vue en coupe schématique d'un oeil humain 9. Les informations lumineuses pénètrent dans l'oeil par la cornée 94, traversent l'humeur aqueuse 95, le cristallin 93, l'humeur vitrée 96, avant d'atteindre la rétine 91 qui contient des récepteurs photosensibles. Une zone aveugle 98 est définie au niveau du nerf optique 97.

La vision humaine s'étend sur une large région d'environ 120° d'angle visuel en vision binoculaire. Cependant, l'homme ne perçoit pleinement les détails et les couleurs que sur une partie réduite de son champ visuel, appelée région fovéale. La région fovéale (zone de la rétine 91 illustrée par la surépaisseur 92) s'étend selon un angle de demi-ouverture compris entre 1 et 2°. Au-delà de la région fovéale, la densité de photorécepteurs qui couvre la rétine humaine décroit fortement, tout d'abord dans une région parafovéale qui s'étend jusqu'à un angle de demi-ouverture d'environ 5°, puis dans une région périphérique pour un angle de demi-ouverture supérieur à 5°.

Un exemple de diagramme de densité de cellules photoréceptrices de l'oeil humain est illustré à la figure 2. On a représenté en ordonnée l'acuité visuelle relative, et en abscisse l'angle par rapport à la région fovéale. La vision humaine, présente un pouvoir de discrimination des détails relativement médiocre au-delà de la région fovéale, comme si la scène observée était floue.

Malgré cela, la perception humaine de l'environnement est colorée et très détaillée. En effet, pour appréhender l'espace environnant, les yeux sont en mouvement permanent pour capturer de multiples images. Ces différentes images sont ensuite assemblées par le cerveau. L'image globale dont l'humain a conscience est ainsi en réalité le résultat de l'intégration de l'exploration permanente de l'environnement par le système oculomoteur. L'enregistrement des mouvements de l'oeil sont ainsi très informatifs concernant un éventuel dysfonctionnement cognitif.

En particulier, malgré la décroissance rapide de la densité de photorécepteurs sur la rétine, le système oculomoteur humain est capable d'extraire un grand nombre d'informations de la région périphérique pour guider le comportement d'exploration oculomoteur.

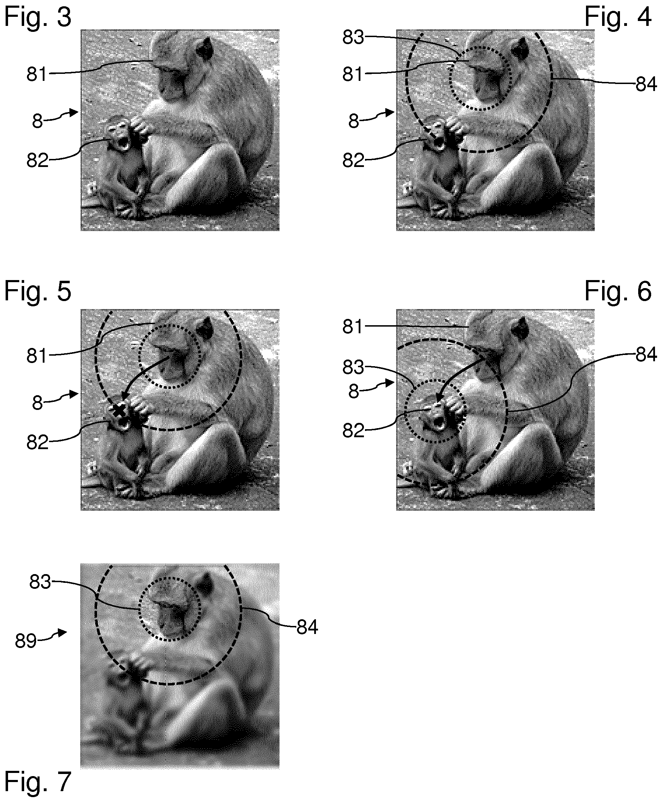

La figure 3 illustre un exemple d'image 8 comprenant plusieurs points d'intérêt. Les principaux points d'intérêt sont ici le visage 81 d'un singe adulte et le visage 82 d'un jeune singe. Les figures 4 à 6 illustrent un exemple de mouvement oculaire en fonction de ces points d'intérêt.

Sur la figure 4, l'image 8 est présentée à un sujet. L'attention et le regard du sujet sont captés par le visage 81 du singe adulte. La vision du sujet est claire et détaillée dans une région 83 incluant le visage 81, et correspondant à la région fovéale. La région 83 est entourée par la région parafovéale 84, dans laquelle la résolution visuelle est nettement moins importante. La vision périphérique à l'extérieur de la région parafovéale 84 a une résolution encore moins importante. La figure 7 illustre la perception 89 de l'image 8 de la figure 3 par le sujet, lorsque la région fovéale 83 est centrée sur le visage 81. La région fovéale 83 s'avère nette, la région parafovéale 84 est perçue modérément floutée, et la région périphérique est perçue plus fortement floutée.

Sur la figure 5, en se basant sur la formation haute résolution de la région fovéale 83 et sur l'information basse résolution de la vision périphérique, le sujet est capable de déterminer le visage 82 du jeune singe comme autre point d'intérêt dans l'image 8.

Comme illustré par la figure 6, afin d'obtenir une vision détaillée de cet autre point d'intérêt, un mouvement oculaire rapide, appelé saccade, permet de déplacer la région fovéale 83 vers le visage 82.

L'invention vise à afficher sur un écran tactile une image dégradée, correspondant par exemple à la figure 7, à partir d'une image mémorisée correspondant par exemple à la figure 3. L'image dégradée vise à reproduire la dégradation d'acuité visuelle entre la région fovéale et la région périphérique.

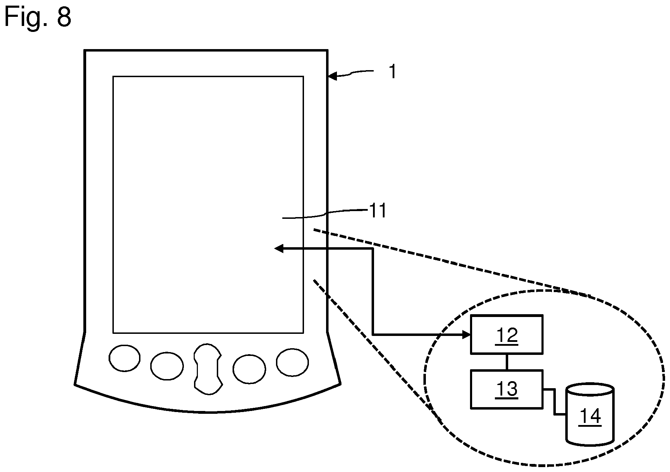

Un exemple de dispositif de détermination de mouvements oculaires 1, pour la mise en oeuvre de l'invention, est illustré à la figure 8. Le dispositif 1 est par exemple mis en oeuvre sous la forme d'un téléphone mobile ou d'une tablette tactile. Le dispositif 1 comprend un afficheur tactile 11. Le dispositif 1 comporte une mémoire numérique 14, mémorisant une ou plusieurs images. Chaque image mémorisée comporte un ou plusieurs points d'intérêt. Les images peuvent être fournies par un serveur distant et n'être mémorisées que transitoirement dans la mémoire numérique 14. Le dispositif 1 comprend par ailleurs un dispositif de traitement 13, par exemple mis en oeuvre sous la forme d'un processeur piloté par une application logicielle. Le dispositif de traitement 13 est susceptible de lire les images mémorisées dans la mémoire numérique 14. Le dispositif 1 comprend par ailleurs une interface de communication 12, pour piloter l'affichage de l'afficheur tactile 11 et pour récupérer les coordonnées d'un point de contact avec l'afficheur tactile 11. L'interface de communication est connectée au dispositif de traitement 13. Un dispositif 1 présentant une structure matérielle d'usage courant peut donc être utilisée, ce qui permet de mettre en oeuvre l'invention avec un dispositif présentant un coût de revient réduit.

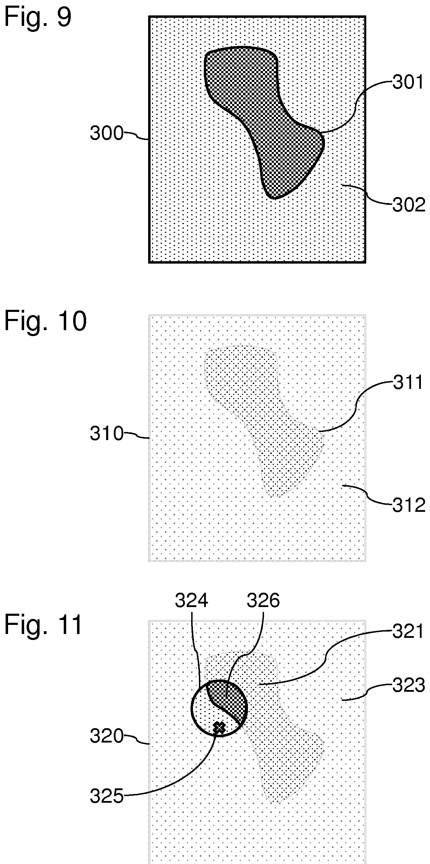

La figure 9 illustre un exemple simplifié d'une image 300 mémorisée dans le dispositif de stockage 14 du dispositif. L'image 300 comporte un point d'intérêt 301. Le point d'intérêt 301 présente par exemple une surface réduite et un contraste lumineux ou coloré par rapport à un fond 302 de l'image 300. Dans l'exemple illustré, le point d'intérêt 301 est une surface à l'intérieur d'une ligne courbe, plus sombre et plus petite qu'un fond 302 en pointillés. L'image 300 est ici uniquement fournie à titre illustratif, toute autre image comportant un ou plusieurs points d'intérêt de façon connue en soi de l'homme du métier, pouvant également être utilisée.

La figure 10 illustre une image intégralement dégradée 310, générée à partir de l'image 300 mémorisée. Par intégralement dégradée, on entend que l'image 310 est dégradée sur toute sa surface. L'image dégradée 310 est ici un cas particulier généré par floutage de l'intégralité de la surface de l'image 300. L'image intégralement dégradée 310 comporte ainsi une zone floutée 311 correspondant au floutage du point d'intérêt 301 de l'image 300, et une zone floutée 312 correspondant au floutage du fond 302.

L'image intégralement dégradée 310 peut être la première image affichée sur l'afficheur tactile 11 pour déterminer l'exploration visuelle au moyen de mesures de l'exploration tactile. Cette image 310 peut ainsi servir d'initiation de l'exploration tactile, le sujet pouvant distinguer la zone floutée 311 correspondant au point d'intérêt 301. Le premier point de contact du sujet sera donc très probablement positionné sur la zone floutée 311.

La figure 11 illustre une image partiellement dégradée 320, générée à partir de l'image 300 mémorisée. L'image partiellement dégradée 320 comporte une zone nette 324 dans laquelle l'image 320 est sensiblement identique à l'image mémorisée 300, en particulier, la zone 326 correspond à la partie du point d'intérêt 301 présente dans la zone nette 324. On détermine la position du dernier point de contact 325 par rapport à l'image en cours d'affichage. La zone nette 324 est positionnée pour inclure la position du dernier point de contact 325 déterminé et ses alentours. Pour des raisons d'ergonomie, la zone nette 324 est ici positionnée au-dessus du point de contact 325, de sorte que la zone nette 324 n'est pas masquée par le doigt du sujet. On va par la suite décrire un point de contact 325 défini avec le doigt du sujet, on peut également envisager un point de contact avec un stylet ou tout autre dispositif de pointage.

L'image partiellement dégradée 320 comporte une zone dégradée 323 en périphérie de la zone nette 324. La zone dégradée 321 correspondant à la zone floutée 311 du point d'intérêt 301 présente dans la zone dégradée 323. La dégradation de la zone 324 permet à la fois :
- d'afficher des informations concernant d'autres zones du point d'intérêt 301, correspondant à une vision périphérique, de sorte que cet affichage suscite un comportement exploratoire pour découvrir ces informations ;
- d'obliger le sujet à réaliser une exploration tactile vers la zone dégradée 323 pour découvrir ces informations, de sorte que le déplacement tactile correspond bien à une exploration oculaire. Si l'image périphérique est nette, l'utilisateur pourrait réaliser une exploration visuelle de l'image affichée, sans déplacer son doigt.

La zone 323 vise par exemple à rendre non perceptibles les microstructures de l'image, tout en conservant les macrostructures. En effet, les macrostructures d'une image affichée sont détectables en vision périphérique et suscitent une exploration visuelle de cette image.

En pratique, le dispositif 1 peut mettre en oeuvre le procédé suivant :
- le lancement de la détermination des mouvements oculaires en affichant l'image 310 sur l'afficheur tactile 11 ;
- dès la détermination d'un point de contact 325 avec l'afficheur tactile 11, la localisation de la position correspondante par rapport à l'image 310. Cette détermination du premier contact est éventuellement le déclencheur d'une temporisation pour limiter la durée du procédé ;
- la génération d'une première image dégradée 320 à partir de l'image mémorisée 300, avec la zone nette 324 incluant la position du point de contact 325 déterminée et ses alentours, avec la zone dégradée 323 correspondant à la dégradation de l'image 300 avec un paramètre de dégradation périphérique ;
- la répétition des étapes successives suivantes :
   - la détermination de la position du dernier point de contact 325 avec l'afficheur tactile 11 et la localisation de la position correspondante dans l'image dégradée en cours d'affichage ;
   - l'enregistrement de la position du dernier point de contact 325 avec l'afficheur tactile 11 ;
   - la génération d'une nouvelle image dégradée 320 à partir de l'image mémorisée 300, la nouvelle image dégradée étant sensiblement identique à l'image mémorisée 300 dans la zone nette 324 qui inclut la position du dernier point de contact 325 et ses alentours, avec la zone dégradée 323 correspondant à la dégradation de l'image 300 avec le paramètre de dégradation périphérique ;
   - l'affichage sur l'afficheur tactile 11 de la nouvelle image dégradée 320 ;
   - la vérification si une condition de fin de test est atteinte (par exemple si la durée d'exploration tactile a atteint un seuil prédéterminé ou si la distance d'exploration tactile a atteint un seuil prédéterminé).

La dégradation entre une image mémorisée 300 et une image dégradée 320 peut être déterminée au moyen d'un indice SSIM, selon le procédé décrit dans la publication intitulée 'Image Quality Assessment: From Error Visibility to Structural Similarity', publiée par Mrs Zhou Wang et alias, le 4 Avril 2004 dans 'IEEE transactions on image Processing' volume 13 numéro 4.

La zone nette 324 de l'image 320 est sensiblement identique à la même zone de l'image mémorisée 300. On peut par exemple considérer que la zone nette 324 de l'image dégradée 320 est sensiblement identique à la zone correspondante de l'image mémorisée 300 si l'indice SSIM moyen calculé entre ces deux zones est au moins égal à 0,9.

On peut également considérer que la zone 323 utilise un paramètre de dégradation optimal correspondant à la vision périphérique, si l'indice SSIM moyen calculé entre la zone floutée 311 et le point d'intérêt 301 (ou entre la zone de point d'intérêt 321 de la zone 323 et la zone correspondante du point d'intérêt 301 dans l'image 300)est avantageusement compris entre 0,4 et 0,8, de préférence entre 0,4 et 0,7, par exemple entre 0, 45 et 0,65 pour favoriser l'exploration visuelle. Une telle dégradation garantit notamment que la région périphérique comportera suffisamment d'informations pour susciter une exploration visuelle et tactile, et pas une quantité d'informations excessive, de sorte que la région périphérique soit bien représentative de la baisse d'acuité visuelle correspondante.

Un indice SSIM moyen trop bas retire trop d'informations de la zone 323 pour susciter une exploration optimale. Un indice SSIM moyen trop élevé conserve trop d'informations dans la zone 323, pour que le sujet déplace son doigt suivant une exploration fidèle à son exploration oculaire.

L'indice SSIM moyen entre le point d'intérêt 301 et la zone dégradée 311, sera désigné par la suite par l'indice SSIMm. L'indice SSIMm sera par exemple calculé comme suit pour un ensemble de points d'intérêt 301 :
- un ensemble de régions d'intérêt 301, constitué de zones saillantes, est sélectionné dans l'image 300. Par exemple, dans le cas d'une photographie, il s'agit des régions sur lesquels la mise au point est faite. Les régions floues et/ou uniformes ne présentent généralement que peu d'intérêt pour le sujet effectuant une exploration visuelle. Des zones floutées 311 de l'image 310 correspondent à ces régions d'intérêt 301 ;
- les régions d'intérêt 301 et les zones floutées 311 sont converties en un nombre fini de niveaux de luminance, par exemple 256 ;
- un indice SSIM est calculé avec des paramètres K1=0,01 et K2=0,03, de façon locale à l'aide d'une fenêtre de pondération gaussienne, symétrique et circulaire d'une taille de 11 x 11 pixels et d'écart type 1,5 pixels ;
- la carte des indices SSIM Iₛₛᵢₘ obtenue est interpolée aux dimensions des régions d'intérêt 301 et des zones floutées 311. On obtient ainsi une valeur d'indice SSIM par pixel. ;
- la valeur moyenne SSIMm des valeurs d'indices SSIM est calculée dans les régions d'intérêt.

Avantageusement, la surface de la zone nette 324 est suffisamment réduite pour que les mouvements du doigt du sujet soient fidèles à ses mouvements oculaires. Si on désigne par S1 la surface de la zone nette 324 et par S2 la surface de l'image dégradée 320 telle qu'affichée sur l'afficheur tactile 11, le rapport S1/S2 est de préférence au plus égal à 0,5, voire au plus égal à 0,15, de sorte que l'exploration visuelle nécessite un déplacement du point de contact du doigt. De préférence, S1 est au plus égale à 60 cm², et de préférence au plus égale à 25 cm². Au-delà de ces dimensions, on pourra considérer que la zone nette 324 présente une surface trop importante par rapport à la vision fovéale.

Le rapport S1/S2 est de préférence au moins égal à 0,01, voire au moins égal à 0,02, de sorte que la zone nette 324 est suffisamment discernable par le sujet, et de sorte que l'adaptation visuelle n'est pas excessivement pénible pour le sujet. Pour un enfant, ou pour analyser un texte avec un stylet, le rapport S1/S2 peut être inférieur. Une telle dimension de la zone nette 324 est également adaptée pour un afficheur tactile 11 de taille réduite avec une résolution relativement basse. De préférence, S1 est au moins égale à 2 cm², et de préférence au moins égale à 3 cm². La zone nette 324 inclut de préférence un cercle d'un diamètre d'au moins 8 mm au-dessus du point de contact 325, de préférence d'au moins 12mm.

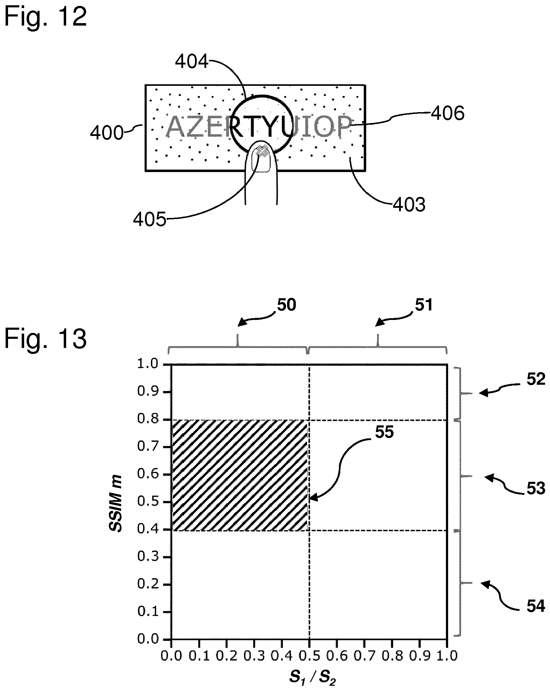

La figure 13 est un diagramme illustrant schématiquement des paramètres garantissant une exploration oculaire optimale par l'intermédiaire du dispositif. Le diagramme fournit le rapport S1/S2 en abscisse, et le paramètre SSIMm en ordonnée (entre le point d'intérêt 301 et la zone floutée 311). La zone correspondant à la référence 55 permet une exploration visuelle optimale avec une bonne fidélité entre le déplacement du doigt et les mouvements oculaires. La zone 50 correspond à l'exigence de surface maximale de la zone nette 324 induisant une exploration tactile fidèle à l'exploration visuelle. La zone 51 correspond à une taille excessive de la zone nette 324 pour que l'exploration tactile corresponde à l'exploration visuelle de façon fidèle. La zone 52 correspond une dégradation insuffisante dans la zone 323 pour conduire à une exploration tactile fidèle à l'exploration visuelle. La zone 53 correspond à une dégradation appropriée pour supprimer les microstructures du point d'intérêt 301, tout en conservant des structures principales. Une telle dégradation s'avère appropriée pour solliciter une exploration tactile. La zone 54 correspond à une dégradation excessive de l'image dans la zone 323, correspondant par exemple à une perte des macrostructures de la zone 321. Une telle dégradation excessive altère à la fois l'exploration visuelle et l'exploration tactile.

Une très grande variété de procédés de dégradation d'image peut être utilisée. On peut notamment envisager une dégradation de l'image mémorisée 300 pour générer la zone dégradée 323, par application d'un flou gaussien, par application d'un filtre moyen, par application d'un sous-échantillonnage, par encodage JPEG avec un taux de compression très élevé.

La figure 12 illustre schématiquement une image partiellement dégradée 400, dans laquelle les points d'intérêt de l'image mémorisée sont des caractères alphanumériques. L'image 400 comporte une zone nette 404 incluant un point de contact 405 du sujet avec l'afficheur tactile. On distingue des caractères alphanumériques nets à l'intérieur de la zone 404. A l'extérieur de la zone nette 404, on trouve une zone dégradée 403 comportant des caractères alphanumériques dégradés 406. Les caractères 406 sont ici déchiffrables mais la dégradation appliquée en pratique est avantageusement réalisée de sorte à les rendre localisables mais non lisibles, afin de susciter une exploration. La dimension de la zone nette 404 peut être adaptée à la dimension des caractères alphanumériques, la zone nette pouvant par exemple inclure entre 1 et 20 caractères alphanumériques en entier. La taille de la zone nette 404 peut être paramétrable, par exemple pour permettre de solliciter le sujet et l'inciter à lire un texte ;

Des tests ont été effectués pour confirmer l'excellente corrélation entre l'exploration oculaire mesurée par oculométrie, et l'exploration tactile mesurée par un procédé selon l'invention. Pour cela, l'exploration tactile avec un procédé de détermination de mouvement oculaire selon l'invention a été comparée à une exploration oculaire effectuée avec un dispositif d'oculométrie de référence.

Les paramètres de l'expérience d'exploration oculaire de référence, mesurée par oculométrie, sont les suivants. Cette expérience d'exploration oculaire de référence a été réalisée avec un système distribué sous la référence commerciale TOBII série 50, associé au logiciel de prétraitement des données distribué sous la référence commerciale Clearview. Des tests ont été réalisés sur 5 sujets sains.

Le protocole de présentation de plusieurs images de test a suivi la procédure suivante :
- 1- Affichage d'une croix de fixation sur un écran, puis attente du déclenchement de l'expérience par le sujet par l'intermédiaire d'un bouton ;
- 2- Affichage préalable d'une croix de fixation pendant 1s sur l'écran ;
- 3- Affichage et exploration libre d'une image test non floutée pendant 6s.
- 4- Retour à l'étape 1.

Un total de 66 images ont été présentées à chaque sujet sur un écran de résolution 1280x1024 pixels, à une distance oeil-écran de 60cm (soit env. 40 pixels/degré d'angle visuel). La résolution de chaque image a été optimisée pour un affichage sans modification sur des écrans de résolution 1280x1024 et 1366x768.

Les stimuli ou points d'intérêts ont été sélectionnés pour inclure deux séries d'images représentant des scènes sociales humaines (32 images) ou de singes de différentes espèces (34 images).

Des scènes sociales humaines ont été sélectionnées pour plusieurs raisons:
- une bonne compréhension d'une scène sociale nécessite un comportement d'exploration oculaire spécifique, prévisible et facilement quantifiable ;
- une exploration oculaire optimale d'une scène sociale nécessite un fonctionnement cognitif intact ;
- une exploration oculaire déficitaire d'une scène sociale peut mettre en avant un trouble cognitif potentiellement handicapant ;
- des difficultés de compréhension des scènes sociales avérées sont présentes dans de nombreuses pathologies neurologiques et psychiatriques (Trouble envahissant du développement, autisme, schizophrénie, démence, etc.).

Les mouvements oculaires ont été modélisés par un modèle de saccade/fixations par le logiciel de prétraitement. Seules les fixations d'une durée égale ou supérieure à 100ms ont été retenues dans les analyses. Pour chaque sujet et chaque image, une carte de chaleur est estimée par noyau (ou méthode de Parzen-Rozenblatt) à l'aide d'un noyau gaussien de variance 500 pixels (σ ≈ 22.4 pixels ≈ 0.5° d'angle visuel) pondéré par le temps de fixation. Enfin, une carte de chaleur est calculée pour chaque stimulus comme la moyenne des cartes de chaleur individuelles normalisées.

Les cartes de chaleurs obtenues sont bien caractéristiques de l'exploration oculaire de scènes sociales. Les différents protagonistes des scènes sociales sont bien détectés par les sujets sains. De façon usuelle, les sujets ont tendance à focaliser la région des yeux le cas échéant.

La détermination des mouvements oculaires par l'intermédiaire de l'exploration tactile a été mise en oeuvre comme suit. Chaque image a été affichée sur un écran tactile d'une résolution de 1366 par 768 pixels. Pour chaque image mémorisée, on a généré une image dégradée, pour simuler la décroissance de résolution spatiale entre la vision fovéale, la vision parafovéale, et la vison périphérique. Cette simulation de décroissance de résolution spatiale est réalisée en conservant une zone nette au niveau du point de contact et ses alentours pour simuler la vision fovéale. La zone nette a été dimensionnée pour correspondre à un angle de demi-ouverture de 2° par rapport à l'oeil du sujet, soit un cercle d'un rayon de 80 pixels. La zone nette donc fovéale, et la zone parafovéale sont générées par application d'une fenêtre gaussienne d'un écart type de 2° ou 80 pixels dans cet exemple. La vision périphérique est simulée par application d'un flou gaussien avec un écart type de 1° à l'extérieur de la zone parafovéale.

Pour des raisons d'ergonomie, la zone nette est positionnée dans l'image dégradée, dans une position légèrement supérieure au point de contact déterminé avec l'écran tactile (25 pixels). L'information colorimétrique a été intégralement conservée sur l'image dégradée.

L'exploration tactile a été enregistrée avec les paramètres suivants :
- les coordonnées de chaque point de contact en abscisse et ordonnée ;
- la durée du contact continu sur ce point de contact.

On peut déterminer une pseudo-fixation oculaire lorsque le contact sur un point de contact est maintenu pendant une durée supérieure à une période d'échantillonnage Tr. Chaque pseudo-fixation peut être enregistrée, avec sa position et sa durée de contact.

Si les points de contact changent continuellement, les coordonnées successives des points de contact sont enregistrées avec des intervalles correspondant à la période d'échantillonnage Tr.

La valeur de la période d'échantillonnage Tr peut être fixée par un compromis entre la résolution temporelle nécessaire à un bon enregistrement du mouvement tactile, et les capacités tactiles du dispositif d'enregistrement. Une période d'échantillonnage Tr comprise entre 20 et 30 ms s'est avérée appropriée, on peut aussi envisager une période d'échantillonnage comprise entre 10 et 50 ms par exemple. Pour un dispositif présentant une vitesse de rafraichissement de l'image faible et/ou une capacité de traitement élevée, on peut utiliser des valeurs de périodes d'échantillonnage beaucoup plus basses.

La mesure des points de contact des sujets avec l'écran tactile a bien permis de caractériser que le mouvement du doigt sur l'image dégradée correspond à un comportement d'exploration visuelle avec une très grande corrélation.

L'exploration tactile selon l'invention révèle des mouvements du doigt plus lents que les mouvements de l'oeil mesurés par oculométrie. Les temps d'exploration respectifs de chaque image ont été adaptés pour les deux types d'exploration, pour les rendre comparables. On peut par exemple interrompre l'exploration tactile après un temps prédéterminé supérieur à un temps prédéterminé utilisé en oculométrie. La durée d'exploration tactile est avantageusement au moins égale à 2 secondes, par exemple au moins 5 secondes.

On peut également envisager de délimiter la durée d'exploration tactile en l'interrompant seulement lorsqu'un seuil de distance d'exploration tactile a été franchi. Ce seuil de distance peut par exemple être exprimé en un nombre de pixels ou en un angle de demi-ouverture. La distance d'exploration tactile peut être déterminée à partir d'une distance moyenne d'exploration visuelle parcourue par un groupe de sujets pendant une durée de 6 secondes. Dans les tests réalisés, cette distance a par exemple été estimée à 2500 pixels par image, avec un écart type approximatif de 500 pixels.

Pour chaque sujet et chaque image, on a calculé un indice de vitesse pour chaque pseudo-fixation (excepté la première). Ce calcul a été réalisé en divisant la distance parcourue entre deux pseudo-fixations successives (d'indice n-1 et n par exemple), par la durée de la dernière de ces deux pseudo-fixations (celle d'indice n dans cet exemple).

Pour chaque image, on peut envisager d'exclure les pseudo-fixations très rapides de l'analyse, en vue d'optimiser les temps de calculs. On peut en effet estimer que des pseudo-fixations très rapides correspondent à des pseudo-saccades, peu porteuses d'informations. Pour chaque image, on peut par exemple envisager d'exclure de l'analyse les 20% les plus rapides des pseudo-fixations.

Comme pour l'oculométrie, on a estimé une carte de chaleur par noyau (ou méthode de Parzen-Rozenblatt) pour chaque sujet et chaque image, à l'aide d'un noyau gaussien de variance 500 pixels (σ ≈ 22.4 pixels ≈ 0.5° d'angle visuel) pondéré par le temps de pseudo-fixation. Enfin, une carte de chaleur est calculée pour chaque stimulus comme la moyenne des cartes de chaleur individuelles normalisées. Une carte de chaleur est calculée pour chaque stimulus comme la moyenne de chacune des cartes de chaleur de chaque sujet pour cette image.

Par comparaison des cartes de chaleur obtenues par oculométrie et par exploration tactile, on a constaté une très grande similitude. On a notamment constaté que les cartes de chaleurs reflétaient bien des pseudo-fixations sur les mêmes points d'intérêt d'une image selon les deux procédés.

On a utilisé un premier indice quantitatif sur les cartes de chaleur obtenues selon les deux procédés. Ce premier indice est une simple mesure de corrélation entre les cartes de chaleur obtenues par les deux procédés. Des valeurs de corrélation comprises entre 0,341 et 0,849 ont été constatées pour des images de scènes sociales humaines. Des valeurs de corrélation comprises entre 0,385 et 0,893 ont été constatées pour des images de scènes sociales de singes. De telles valeurs de corrélation s'avèrent déjà suffisamment élevées pour garantir la fidélité de l'exploration tactile par rapport à l'exploration oculaire. De façon générale, on favorisera des images pour les tests, illustrant plusieurs êtres vivants, afin d'inclure des scènes sociales, en particulier pour des applications d'identification de troubles.

L'exploration tactile selon l'invention est donc fidèle à l'exploration oculaire, notamment du fait que l'exploration tactile utilise une approche biomimétique, en simulant les traitements effectués au niveau de la rétine du sujet.

La variabilité des scores de corrélation semble plus en relation avec la complexité de l'image testée qu'avec une réelle différence de comportements d'exploration entre les deux procédés. En effet, plus une image est complexe, plus le sujet à tendance à réaliser des mouvements aléatoires dans sa stratégie de découverte d'une image.

## Revendications

1. Dispositif (1) pour la détermination de mouvements oculaires, comprenant:
- un afficheur tactile (11) ;
- une mémoire numérique (14) mémorisant une image (300) incluant au moins un point d'intérêt (301) ;
- un dispositif de traitement (13), configuré pour mettre en oeuvre une répétition d'étapes de :
- détermination de la position d'un point de contact (325) d'un utilisateur avec l'afficheur tactile (11) et localisation de la position correspondante dans l'image en cours d'affichage (320) sur l'afficheur tactile ;
- génération d'une image dégradée (320) à partir de l'image mémorisée, l'image dégradée étant :
- sensiblement identique à l'image mémorisée dans une zone nette (324) incluant la position du point de contact déterminée et ses alentours ;
- dégradée avec un paramètre de dégradation périphérique au-delà de la zone nette ;
- affichage sur l'afficheur tactile (11) de l'image dégradée générée (320) ;
- l'enregistrement des positions successives du point de contact (325) avec l'afficheur tactile (11).

2. Procédé de détermination de mouvements oculaires, comprenant :
- la fourniture d'un dispositif (1) incluant un afficheur tactile (11) et une mémoire numérique (14) mémorisant une image (300) incluant au moins un point d'intérêt (301) ;
- la répétition d'étapes de :
- détermination de la position d'un point de contact (325) d'un utilisateur avec l'afficheur tactile (11) et localisation de la position correspondante dans une image en cours d'affichage (320) sur l'afficheur tactile ;
- génération d'une image dégradée (320) à partir de l'image mémorisée, l'image dégradée étant :
- sensiblement identique à l'image mémorisée dans une zone nette (324) incluant la position du point de contact (325) déterminée et ses alentours ;
- dégradée (323) avec un paramètre de dégradation périphérique au-delà de la zone nette ;
- affichage sur l'afficheur tactile de l'image dégradée générée (320) ;
- l'enregistrement des positions successives du point de contact avec l'afficheur tactile.

3. Procédé de détermination de mouvements oculaires selon la revendication 2, dans lequel ladite image dégradée (320) est générée par application d'un floutage de l'image mémorisée (300) au-delà de la zone nette (324).

4. Procédé de détermination de mouvements oculaires selon la revendication 2 ou 3, dans lequel la génération de l'image dégradée (320) à partir de l'image mémorisée est configurée pour qu'un indice SSIM moyen entre l'image mémorisée (300) et l'image dégradée (320) soit compris entre 0,4 et 0,8 et dans lequel le rapport entre la surface de la zone nette (324) et la surface de l'image dégradée générée (320) est compris entre 0,01 et 0,5.

5. Procédé de détermination de mouvements oculaires selon la revendication 4, dans lequel la génération de l'image dégradée (320) à partir de l'image mémorisée est configurée pour qu'un indice SSIM moyen entre l'image mémorisée (300) et l'image dégradée (320) soit compris entre 0,4 et 0,7 et dans lequel le rapport entre la surface de la zone nette (324) et la surface de l'image dégradée générée (320) est compris entre 0,01 et 0,15.

6. Procédé de détermination de mouvements oculaires selon l'une quelconque des revendications 2 à 5, dans lequel ladite zone nette (324) inclut un cercle d'un diamètre d'au moins 8 mm au-dessus du point de contact (325).

7. Procédé de détermination de mouvements oculaires selon l'une quelconque des revendications 2 à 6, dans lequel la position du point de contact (325) de l'utilisateur avec l'afficheur tactile (11) est échantillonnée avec une période comprise entre 10 et 50 ms.

8. Procédé de détermination de mouvements oculaires selon l'une quelconque des revendications 2 à 7, dans lequel ladite répétition d'étapes est exécutée pendant une durée au moins égale à 5 secondes.

9. Procédé de détermination de mouvements oculaires selon l'une quelconque des revendications 2 à 8, dans lequel l'image mémorisée (300) illustre plusieurs êtres vivants formant chacun au moins un point d'intérêt.

10. Procédé de détermination d'une anomalie d'exploration visuelle, comprenant
- l'exécution d'un procédé de détermination de mouvements oculaires selon l'une quelconque des revendications 2 à 9 ;
- la comparaison entre les positions successives enregistrées du point de contact et une trajectoire d'exploration visuelle de référence ;
- la détermination d'une anomalie d'exploration visuelle lorsque les positions successives enregistrées du point de contact divergent de la trajectoire d'exploration visuelle de référence au-delà d'un certain seuil.

## Patentansprüche

1. Vorrichtung (1) zur Bestimmung der Augenbewegungen, umfassend:
- einen Berührungsbildschirm (11);
- einen digitalen Speicher (14), der ein Bild (300) speichert, das mindestens einen Punkt von Interesse (301) umfasst;
- eine Behandlungsvorrichtung (13), die ausgelegt ist, eine Wiederholung von Schritten durchzuführen:
- Bestimmen der Position eines Kontaktpunkts (325) eines Bedieners mit dem Berührungsbildschirm (11) und Lokalisieren der entsprechenden Position in dem Bild während der Anzeige (320) auf dem Berührungsbildschirm;
- Generieren eines degradierten Bilds (320) aus dem gespeicherten Bild, wobei das degradierte Bild:
- mit dem gespeicherten Bild in einer klaren Zone (324) im Wesentlichen identisch ist, welche die Position des bestimmten Kontaktpunkts und seiner Umgebung umfasst;
- mit einem peripheren Degradationsparameter außerhalb der klaren Zone degradiert ist;
- Anzeigen des generierten degradierten Bilds (320) auf dem Berührungsbildschirm (11);
- Aufzeichnen aufeinanderfolgender Positionen des Kontaktpunkts (325) mit dem Berührungsbildschirm (11).

2. Verfahren zur Bestimmung der Augenbewegungen, umfassend:
- Bereitstellen einer Vorrichtung (1), die einen Berührungsbildschirm (11) und einen digitalen Speicher (14) umfasst, der ein Bild (300) speichert, das mindestens einen Punkt von Interesse (301) umfasst;
- die Wiederholung von Schritten:
- Bestimmen der Position eines Kontaktpunkts (325) eines Bedieners mit dem Berührungsbildschirm (11) und Lokalisieren der entsprechenden Position in einem Bild während der Anzeige (320) auf dem Berührungsbildschirm;
- Generieren eines degradierten Bilds (320) aus dem gespeicherten Bild, wobei das degradierte Bild:
- mit dem gespeicherten Bild in einer klaren Zone (324) im Wesentlichen identisch ist, welche die Position des bestimmten Kontaktpunkts (325) und seiner Umgebung umfasst;
- mit einem peripheren Degradationsparameter außerhalb der klaren Zone degradiert ist (323);
- Anzeigen des generierten degradierten Bilds (320) auf dem Berührungsbildschirm;
- Aufzeichnen aufeinanderfolgender Positionen des Kontaktpunkts (325) mit dem Berührungsbildschirm.

3. Verfahren zur Bestimmung der Augenbewegungen nach Anspruch 2, wobei das degradierte Bild (320) durch Anlegen eines Unkenntlichmachens an das gespeicherte Bild (300) außerhalb der klaren Zone (324) generiert wird.

4. Verfahren zur Bestimmung der Augenbewegungen nach Anspruch 2 oder 3, wobei das Generieren des degradierten Bilds (320) aus dem gespeicherten Bild derart ausgelegt ist, dass ein mittlerer SSIM-Index zwischen dem gespeicherten Bild (300) und dem degradierten Bild (320) zwischen 0,4 und 0,8 liegt, und wobei das Verhältnis zwischen der Fläche der klaren Zone (324) und der Fläche des generierten degradierten Bilds (320) zwischen 0,01 und 0,5 liegt.

5. Verfahren zur Bestimmung der Augenbewegungen nach Anspruch 4, wobei das Generieren des degradierten Bilds (320) aus dem gespeicherten Bild derart ausgelegt ist, dass ein mittlerer SSIM-Index zwischen dem gespeicherten Bild (300) und dem degradierten Bild (320) zwischen 0,4 und 0,7 liegt, und wobei das Verhältnis zwischen der Fläche der klaren Zone (324) und der Fläche des generierten degradierten Bilds (320) zwischen 0,01 und 0,15 liegt.

6. Verfahren zur Bestimmung der Augenbewegungen nach einem der Ansprüche 2 bis 5, wobei die klare Zone (324) einen Kreis mit einem Durchmesser von mindestens 8 mm über dem Kontaktpunkt (325) umfasst.

7. Verfahren zur Bestimmung der Augenbewegungen nach einem der Ansprüche 2 bis 6, wobei die Position des Kontaktpunkts (325) des Bedieners mit dem Berührungsbildschirm (11) mit einer Periode zwischen 10 und 50 ms abgetastet wird.

8. Verfahren zur Bestimmung der Augenbewegungen nach einem der Ansprüche 2 bis 7, wobei die Wiederholung von Schritten während einer Dauer von mindestens gleich 5 Sekunden ausgeführt wird.

9. Verfahren zur Bestimmung der Augenbewegungen nach einem der Ansprüche 2 bis 8, wobei das gespeicherte Bild (300) mehrere Lebewesen darstellt, die jeweils mindestens einen Punkt von Interesse bilden.

10. Verfahren zur Bestimmung einer Anomalie einer visuellen Exploration, umfassend:
- Ausführen eines Verfahrens zur Bestimmung der Augenbewegungen nach einem der Ansprüche 2 bis 9;
- Vergleichen zwischen aufeinanderfolgenden aufgezeichneten Positionen des Kontaktpunkts und einer Bahn einer visuellen Referenzexploration;
- Bestimmen einer Anomalie der visuellen Exploration, wenn die aufeinanderfolgenden aufgezeichneten Positionen des Kontaktpunkts von der Bahn der visuellen Referenzexploration über eine bestimme Schwelle hinaus abweichen.

## Claims

1. Device (1) for the determination of eye movements, comprising:
- a touch display (11);
- a digital memory (14) storing an image (300) including at least one point of interest (301);
- a processing device (13), configured to implement a repetition of steps of:
- determination of the position of a point of contact (325) of a user with the touch display (11) and location of the corresponding position in the image in the course of display (320) on the touch display;
- generation of a degraded image (320) on the basis of the stored image, the degraded image being:
- substantially identical to the stored image in a sharp zone (324) including the determined position of the point of contact and its surroundings;
- degraded with a parameter of peripheral degradation beyond the sharp zone;
- display on the touch display (11) of the degraded image generated (320);
- the recording of the successive positions of the point of contact (325) with the touch display (11).

2. Method for determining eye movements, comprising:
- the provision of a device (1) including a touch display (11) and a digital memory (14) storing an image (300) including at least one point of interest (301);
- the repetition of steps of:
- determination of the position of a point of contact (325) of a user with the touch display (11) and location of the corresponding position in an image in the course of display (320) on the touch display;
- generation of a degraded image (320) on the basis of the stored image, the degraded image being:
- substantially identical to the stored image in a sharp zone (324) including the determined position of the point of contact (325) and its surroundings;
- degraded (323) with a parameter of peripheral degradation beyond the sharp zone;
- display on the touch display of the degraded image generated (320);
- the recording of the successive positions of the point of contact (325) with the touch display.

3. Method for determining eye movements according to Claim 2, in which the said degraded image (320) is generated by application of a blurring of the stored image (300) beyond the sharp zone (324).

4. Method for determining eye movements according to Claim 2 or 3, in which the generation of the degraded image (320) on the basis of the stored image is configured so that a mean index SSIM between the stored image (300) and the degraded image (320) is between 0.4 and 0.8 and in which the ratio of the surface area of the sharp zone (324) to the surface area of the degraded image generated (320) is between 0.01 and 0.5.

5. Method for determining eye movements according to Claim 4, in which the generation of the degraded image (320) on the basis of the stored image is configured so that a mean index SSIM between the stored image (300) and the degraded image (320) is between 0.4 and 0.7 and in which the ratio of the surface area of the sharp zone (324) to the surface area of the degraded image generated (320) is between 0.01 and 0.15.

6. Method for determining eye movements according to any one of Claims 2 to 5, in which the said sharp zone (324) includes a circle of a diameter of at least 8 mm above the point of contact (325).

7. Method for determining eye movements according to any one of Claims 2 to 6, in which the position of the point of contact (325) of the user with the touch display (11) is sampled with a period of between 10 and 50 ms.

8. Method for determining eye movements according to any one of Claims 2 to 7, in which the said repetition of steps is executed for a duration at least equal to 5 seconds.

9. Method for determining eye movements according to any one of Claims 2 to 8, in which the stored image (300) illustrates several living beings each forming at least one point of interest.

10. Method for determining a visual exploration anomaly, comprising:
- the execution of a method for determining eye movements according to any one of Claims 2 to 9;
- the comparison between the recorded successive positions of the point of contact and a reference visual exploration trajectory;
- the determination of a visual exploration anomaly when the recorded successive positions of the point of contact diverge from the reference visual exploration trajectory beyond a certain threshold.
